# EUROPEAN PATENT SPECIFICATION

(11) **EP 1 866 103 B1**
(45) Date of publication and mention of the grant of the patent: **22.07.2009**
(21) Application number: 06726474.7
(22) Date of filing: 24.03.2006
(51) Int. Cl.: B05D 7/24, G01N 33/543, C23C 18/42

(54) **A METHOD FOR PRODUCING AN ALDEHYDE CONTAINING COATING**
VERFAHREN ZUR HERSTELLUNG EINER ALDEHYDHALTIGEN BESCHICHTUNG
MÉTHODE DE PRODUCTION D'UN REVÊTEMENT CONTENANT UN ALDÉHYDE

(30) Priority: 24.03.2005 GB 0506051
(43) Date of publication of application: 19.12.2007
(73) Proprietor: Surface Innovations Limited, Wolsingham, County Durham DL13 3HH (GB)
(72) Inventor: BADYAL, Jas, Pal, Singh, Wolsingham, County Durham DL13 3HH (GB); SCHOFIELD, Wayne, Christopher, Edward, Marlston-cum-Lache, Chester CH4 8HH (GB); MCGETTRICK, James, Durham City DH1 3HP (GB)
(74) Representative: Tomkinson, Alexandra
(86) International application number: PCT/GB2006/001052
(87) International publication number: WO 2006/100480

(56) References cited:
- WO-A-00/44207
- US-A1- 2004 086 660
- US-B1- 6 528 291
- BEUMER, G. J.; GONG, X.; DAI, L.; ST. JOHN, H. A. W.; GRIESSER,H. J.: "Aldehyde Plasma Polymers" POLYMER PREPRINTS, vol. 38, 1997, pages 1037-1038, XP009068331 ACS Polym. Div.
- LEICH M A ET AL: "PULSED PLASMA POLYMERIZATION OF BENZALDEHYDE FOR RETENTION OF THE ALDEHYDE FUNCTIONAL GROUP" MACROMOLECULES, ACS, WASHINGTON, DC, US, vol. 31, no. 22, 3 November 1998 (1998-11-03), pages 7618-7626, XP000788805 ISSN: 0024-9297 cited in the application

## Description

The present invention relates to the production of coatings which contain aldehyde functional groups.

The surface functionalisation of solid objects is a topic of considerable technological importance, since it offers a cost effective means of improving substrate performance without affecting the overall bulk properties. For instance, the attachment of biomolecules such as DNA or proteins is of great technical interest, allowing the construction of biological arrays that are finding application in fields of study as diverse as computing (Aldeman, M. Science 1994, 266, 1021; Frutos, A. G. et al., Nuc. Acids Res. 1997, 25, 4748), drug discovery (Debouck, C. et al., Nature Genet. 1999, 1(suppl.) 48), cancer research (Van't Veer, L. J. et al. Nature 2002, 415, 530) and the elucidation of the human genome (McGlennen, R. C. Clinical Chemistry 2001, 47, 393). In addition, silver can be deposited onto aldehyde surfaces via Tollens reaction to yield anti-bacterial properties (Manolache, S. et al., Journal of Photopolymer Science and Technology 2000, 13, 51; Hongquan, J. et al., J. Appl. Polym. Sci. 2004, 93, 1411).

Furthermore, an aldehyde surface offers a chemically versatile substrate that allows surface modification by the application of widely used solution-based chemistries including, but not limited to, the Aldol reaction; the Canizzarro reaction, the Mannich reaction, the Reformatsky reaction, the Tischenko reaction, the Wittig reaction, benzoin formation, bimolecular reduction to 1,2-diols, reductive alkylation/halogenation, conversion to acyls, anhydrides, γ-keto esters/nitriles or 1,4-diketones, acetals, amides, carboxylic esters, dihalides, epoxides, formats, halo alcohols and ethers, β-keto esters and ketones, ketones, nitriles, oximes, phenols, silyl enol ethers. Further reactions include the acylation of heterocyclic systems, photochemical cleavage, decarbonylation, halogenation, and the oxidation or reduction of the aldehyde functionality.

Aldehydes can also undergo molecular rearrangements to yield ketones (alkyl-interchange reaction) and indole compounds (upon treatment with phenylhydrazine and a catalyst, the Fischer indole synthesis).

Another application of aldehydes is in condensation reactions including, but not limited to, condensation with active hydrogen compounds, anhydrides, aromatic rings, carboxylic esters, halo esters, and phopsphoranes. Aldehydes are also known to react with species including, but not limited to alcohols, alkenes, amines (the Schiff-base reaction), ammonia, carbon dioxide, hydrogen cyanide, hydrazines, ketenes, metalated aldimines, organometallic compounds, sulfamide, sodium bisulfite, thiobenzilic acid, thiols (including hydrogen sulphide) and can undergo selenation or sulfonation (March, J., Advanced Organic Chemistry 4th ed., Wiley-Interscience, New York 1992).

Existing methods of functionalising solid surfaces with aldehyde groups include aldehyde-silane self-assembly (Zammateo, N. et al, Anal. Biochem. 2000, 280, 143), aldehyde-thiol self-assembly, the conversion of surface immobilised epoxide functionalities (Pitt, W. G. et al Journal of Biomedical Materials Research, Part A 2004, 68A, 95), and the immobilisation of aldehyde containing linkers (typically glutaraldehyde) to other functionalised surfaces (Duman, M. et al., Biosensors and Bioelectronics 2003, 18, 1355; Yokoyama et al., WO 2003046562). All of these approaches suffer from drawbacks such as involving multistep processes, substrate specificity, and the requirement for solution phase chemistry.

Another method of forming aldehyde functionality on a surface involves treatment of a polymer surface, such as polyurethane, with a gas plasma, such as carbon dioxide. However, such approaches lead to the generation of a wide range of surface functions such as carboxylic acids or hydroxyl groups (Terlingen, J. G. A. et al., J. Appl, Polym. Sci. 1995, 57, 969).

Surface functionalisation by continuous wave plasma polymerisation is an additional route by which aldehydes have been attached to solid surfaces. This approach suffers from the drawback of poor structural retention, with surfaces showing increased oxygenation and/or a loss of aldehyde functionality compared to their monomer precursors (Baumer et al. European Patent EP 1131359; Chow, et al. US Patent 6528291; Griesser, H. J. et al., Mat Res Soc. Symp. Proc. 1999, 544, 9*;* Gong, X. et al., Journal of Polymer Science B: Polymer Physics 2000, 38, 2323; Chen, Q. et al., J. Phys. Chem. B 2001, 105, 618; McLean, K. M. et al., Colloids and Surfaces B: Biointerfaces 2000, 18, 221).

Plasma polymers are hence often regarded as being structurally dissimilar compared to conventional polymers, since they possess high levels of cross-linking and lack a regular repeat unit (Yasuda, H. Plasma Polymerisation Academic Press: New York, 1985). This can be attributed to the plasma environment generating a whole range of reactive intermediates which contribute to the overall lack of chemical selectivity. However, it has been found that pulsing the electric discharge on the ms-µs timescale can significantly improve structural retention of the parent monomer species (Panchalingam, V. et al., Appl. Polym. Sci. 1994, 54, 123; Han, L. M. et al., Chem. Mater., 1998, 10, 1422; Timmons et al., US patent no. US 5,876,753) and in some cases conventional linear polymers have been synthesised (Han, L. M. et al., J. Polym.Sci., Part A: Polym. Chem. 1998, 36, 3121). Under such conditions, repetitive short bursts of plasma are understood to control the number and lifetime of active species created during the on-period, which then is followed by conventional reaction pathways (e.g. polymerisation) occurring during the off-period (Savage, C. R. et al., Chem. Mater., 1991, 3, 575).

The preparation of aldehyde functionalised surfaces by pulsed plasma polymerisation has been previously reported using benzaldehyde (Leich, M. A. et al., Macromolecules 1998, 31, 7618). However, the retention of monomer structure was poor and the coated surfaces exhibited low levels of usable aldehyde functionality. The observed inadequate level of sample performance was due to the structure of the monomer utilised. Benzadelhyde lacks a functional group, such as an acrylate or alkene functionality, that can be readily polymerised by conventional reaction pathways during the pulsed plasma off-time without damage to the desired aldehyde moiety. Plasma polymerisation of benzaldehyde, even under mild pulsing conditions, must proceed via its aryl group resulting in unavoidable rupture of the monomer structure and potential damage to the neighbouring aldehyde functionality. Hence, to achieve the successful deposition of an aldehyde containing surface, a methodology combining both pulsed plasma techniques and the selection of a suitable polymerisable monomer structure must be utilised.

The applicants have found that pulsed plasma polymerisation of monomers containing aldehyde functionalities of general formula (I) can potentially overcome the limitations of existing techniques for forming aldehyde functionalised surfaces. Compounds of formula (I) possess unsaturated functional groups (such as alkene, acrylate and methacrylate) that can undergo conventional polymerisation pathways during the pulsed plasma off-time with negligible impact on the desired aldehyde moiety. The resulting films, in comparison with the prior art, exhibit almost total retention of monomer functionality and have been found capable of the exacting levels of performance demanded by applications such as DNA microarray production.

According to the present invention there is provided a method for applying a reactive aldehyde containing coating to a substrate, said method including subjecting said substrate to a plasma discharge in the presence of a compound of formula (I):

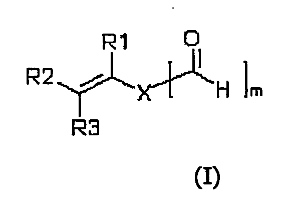

Where X is an optionally substituted straight or branched alkylene chain(s) or aryl group(s); R¹ R² or R³ are optionally substituted hydrocarbyl or heterocyclic groups; and m is an integer greater than 0.

As used herein, the term "hydrocarbyl" includes alkyl, alkenyl, alkynyl, aryl and aralkyl groups. The term "aryl" refers to aromatic cyclic groups such as phenyl or naphthyl, in particular phenyl. The term "alkyl" refers to straight or branched chains of carbon atoms, suitably of from 1 to 20 carbon atoms in length. The terms "alkenyl" and "alkynyl" refer to straight or branched unsaturated chains suitably having from 2 to 20 carbon atoms. These groups may have one or more multiple bonds. Thus examples of alkenyl groups include allenyl and dienyl.

Suitable optional substituents for hydrocarbyl groups R¹, R², R³ and alkylene/aryl groups X are groups that are substantially inert during the process of the invention. They may include halo groups such as fluoro, chloro, bromo and/or iodo. Particularly preferred halo substituents are fluoro.

In a preferred embodiment of the invention, X is a moiety comprising an ester group adjacent to an optionally substituted hydrocarbyl or heterocyclic group, R⁴. Thus, in a particular embodiment, the compound of formula (I) is a compound of formula (II):

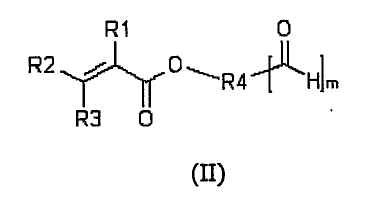

In particular, R¹, R², R³ and R⁴ are independently selected from hydrogen or alkyl, and in particular, from hydrogen or C₁₋₆ alkyl, such as methyl. Thus, in a particularly preferred embodiment, the compound of formula (II) is a compound of formula (III): the desired aldehyde functionality is connected to a readily polymerised acrylate group (CH₂=CH-CO₂-) via a saturated alkyl hydrocarbon chain linker, R⁴, where n is an integer of from 1 to 20:

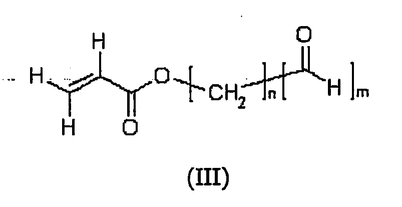

A particular example of a compound of formula (III), where m=1 and n=1, is ethylaldehyde acrylate.

In another particularly preferred embodiment, the compound of formula (II) is a compound of formula (IIIa): the desired aldehyde functionality is connected to a readily polymerised methacrylate group (CH₂=C(CH₃)-CO₂-) via a saturated alkyl hydrocarbon chain linker, R⁴, where n is an integer of from 1 to 20::

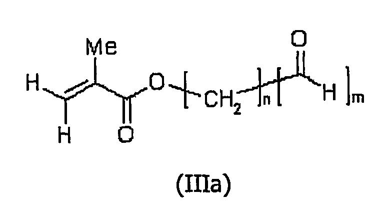

A particular example of a compound of formula (IIIa), where m=1 and where n=1, is ethylaldehyde methacrylate

In other particularly preferred embodiments of the invention, with reference to the compound of formula (I), R¹, R² and R³ are again independently selected from hydrogen or alkyl, and in particular, from hydrogen or C₁₋₆ alkyl, such as methyl. Thus, in another particular embodiment, the compound of formula (I) is a compound of formula (IV): 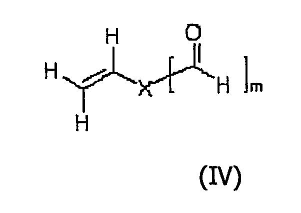 where X is as defined above and m is an integer greater than 0.

Particularly preferred compounds of formula (IV) are vinylbenzenes of formula (V), where X is a di-substituted aromatic ring: 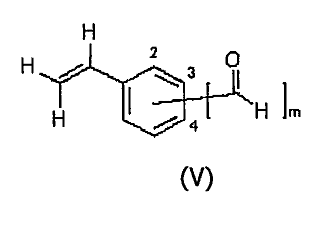 where the ring can be ortho, meta or para substituted.

A particular example of a compound of formula (V) is 3-vinylbenzaldehyde.

In another particularly preferred example of the compound of formula (IV), X is a saturated alkyl hydrocarbon chain. Thus, the compound of formula (IV) is a compound of formula (V) where n is an integer of from 1 to 20, for example from 1 to 10 and preferably 8.

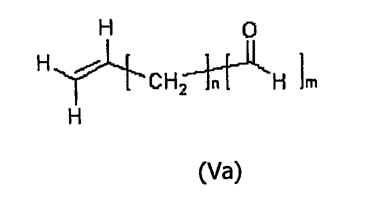

A particular example of a compound of formula (Va), where m=1 and n=8, is 10-undecenal.

Precise conditions under which the pulsed plasma deposition of the compound of formula (I) takes place in an effective manner will vary depending upon factors such as the nature of the monomer, the substrate, the size and architecture of the plasma deposition chamber etc. and will be determined using routine methods and/or the techniques illustrated hereinafter. In general however, polymerisation is suitably effected using vapours or atomised droplets of compounds of formula (I) at pressures of from 0.01 to 999 mbar, suitably at about 0.2 mbar. Although atmospheric-pressure and sub-atmospheric pressure plasmas are known and utilised for plasma polymer deposition in the art.

A glow discharge is then ignited by applying a high frequency voltage, for example at 13.56 MHz. The applied fields are suitably of an average power of up to 50 W.

The fields are suitably applied for a period sufficient to give the desired coating. In general, this will be from 30 seconds to 60 minutes, preferably from 1 to 15 minutes, depending upon the nature of the compound of formula (I) and the substrate etc.

Suitably, the average power of the pulsed plasma discharge is low, for example of less than 0.05 W/cm³, preferably less than 0.025 W/cm³ and most preferably less than 0.0025 W/cm³.

The pulsing regime which will deliver such low average power discharges will vary depending upon the nature of the substrate, the size and nature of the discharge chamber etc. However, suitable pulsing arrangements can be determined by routine methods in any particular case. A typical sequence is one in which the power is on for from 10 µs to 100 µs, and off for from 1000 µs to 20000 µs.

In one embodiment of the invention the pulsing regime is varied during the course of coating deposition so as to enable the production of gradated coatings. For example, a high average-power pulsing regime may be used at the start of sample treatment to yield a highly cross-linked, insoluble sub-surface coating that adheres well to the substrate. A low average-power pulsing regime may then be adopted for conclusion of the treatment cycle, yielding a surface layer displaying high levels of retained monomer aldehyde functionality on top of said well-adhered sub-surface. Such a regime would be expected to improve overall coating durability and adhesion, without sacrificing any of the desired surface properties (i.e. reactive surface aldehyde functionality).

Suitable plasmas for use in the method of the invention include non-equilibrium plasmas such as those generated by audio-frequencies, radiofrequencies (RF) or microwave frequencies. In another embodiment the plasma is generated by a hollow cathode device. In yet another embodiment, the pulsed plasma is produced by direct current (DC).

The plasma may operate at low, sub-atmospheric or atmospheric pressures as are known in the art. The monomer may be introduced into the plasma as a vapour or an atomised spray of liquid droplets (WO03101621 and WO03097245, Surface Innovations Limited). The monomer may be introduced into the pulsed plasma deposition apparatus continuously or in a pulsed manner by way of, for example, a gas pulsing valve

The substrate to which the aldehyde bearing coating is applied will preferentially be located substantially inside the pulsed plasma during coating deposition, However, the substrate may alternatively be located outside of the pulsed plasma, thus avoiding excessive damage to the substrate or growing coating.

The monomer will typically be directly excited within the plasma discharge. However, "remote" plasma deposition methods may be used as are known in the art. In said methods the monomer enters the deposition apparatus substantially "downstream" of the pulsed plasma, thus reducing the potentially harmful effects of bombardment by short-lived, high-energy species such as ions.

The plasma may comprise the monomeric compound alone, in the absence of other compounds or in admixture with for example an inert gas. Plasmas consisting of monomeric compound alone may be achieved as illustrated hereinafter, by first evacuating the reactor vessel as far as possible, and then purging the reactor vessel with the organic compound for a period sufficient to ensure that the vessel is substantially free of other gases. The temperature in the plasma chamber is suitably high enough to allow sufficient monomer in gaseous phase to enter the plasma chamber. This will depend upon the monomer and conveniently ambient temperature will be employed. However, elevated temperatures for example from 25 to 250 °C may be required in some cases.

In alternative embodiments of the invention, materials additional to the plasma polymer coating precursor are present within the plasma deposition apparatus. The additional materials may be introduced into the coating deposition apparatus continuously or in a pulsed manner by way of, for example, a gas pulsing valve.

Said additive materials may be inert and act as buffers without any of their atomic structure being incorporated into the growing plasma polymer (suitable examples include the noble gases). A buffer of this type may be necessary to maintain a required process pressure. Alternatively the inert buffer may be required to sustain the plasma discharge. For example, the operation of atmospheric pressure glow discharge (APGD) plasmas often requires large quantities of helium. This helium diluent maintains the plasma by means of a Penning Ionisation mechanism without becoming incorporated within the deposited coating.

In other embodiments of the invention, the additive materials possess the capability to modify and/or be incorporated into the coating forming material and/or the resultant plasma deposited coating. Suitable examples include other reactive gases such as halogens, oxygen, and ammonia.

In alternative embodiments of the invention, the additive materials may be other monomers. The resultant coatings comprise copolymers as are known and described in the art. Suitable monomers for use within the method of the invention include organic (e.g. styrene), inorganic, organo-silicon and organo-metallic monomers.

The invention further provides a substrate having an aldehyde containing coating thereon, obtained by a process as described above. Such substrate can include any solid, particulate, or porous substrate or finished article, consisting of any materials (or combination of materials) as are known in the art. Examples of materials include any or any combination of, but are not limited to, woven or non-woven fibres, natural fibres, synthetic fibres, metal, glass, ceramics, semiconductors, cellulosic materials, paper, wood, or polymers such as polytetrafluoroethylene, polythene or polystyrene. In a particular embodiment, the surface comprises a support material, such as a polymeric material, used in biochemical analysis.

In one embodiment of the invention the substrate is coated by means of a reel-to-reel apparatus. This coating process can take place continuously. In one embodiment the substrate is moved past and through a coating apparatus acting in accordance with this invention.

The pulsed plasma polymerisation of the invention is therefore a solventless method for functionalising solid surface with aldehyde groups.

Once the aldehyde functional coating has been applied to the substrate, the aldehyde group may be further derivatised as required. In particular, it may be reacted with an amine such as an amine terminated oligonucleotide strand. The derivatisation reaction may be effected in the gaseous phase where the reagents allow, or in a solvent such as water or an organic solvent. Examples of such solvents include alcohols (such as methanol), and tetrahydrofuran.

The derivatisation may result in the immobilisation of an amine containing reagent on said surface. If derivatisation is spatially addressed, as is known in the art, this results in chemical patterning of the surface. A preferred case of an aldehyde surface patterned with amine containing biomolecules is a biological microarray. A particularly preferred case is one in which the amine containing biomolecule is a DNA strand, resulting in a DNA microarray. Another preferred embodiment is one in which the amine containing biomolecule is a protein or fragment thereof, resulting in a protein microarray.

Aldehyde functionalised surfaces produced in accordance with the invention were derivatized with a variety of amine-containing reagents (e. g. oligonucleotide strands, proteins, and derivatized sugars). Furthermore, these aldehyde functionalised surfaces produced in accordance with the invention enabled the construction of DNA microarrays by a procedure shown diagrammatically in Scheme 1.

In one embodiment a solution of silver containing salt reacts with surface aldehyde groups, resulting in silver metallization of the polymer surface. The Tollens reaction can be used to generate metallic silver on the reactive aldehyde surface.

Thus in a further embodiment, the invention provides a method for the immobilisation of an amine containing reagent at a surface, said method including the application of a reactive aldehyde containing coating to said surface by a method described above, and then contacting the surface with a solution of said amine-containing agent under conditions such that the amine-containing agent reacts with the aldehyde groups.

Preferably the amine solution is spatially addressed onto the reactive aldehyde containing surface, such that amine immobilisation occurs only in given spatial locations. The spatial restriction can be achieved by plasma depositing the aldehyde functional coating through a mask or template. This produces a sample exhibiting regions covered with aldehyde functional coating juxtaposed with regions that exhibit no aldehyde functional coating.

Pulsed plasma polymerization in accordance with the invention has been found to be an effective means for functionalizing solid substrates with aldehyde groups. The resulting functionalised surfaces are amenable to conventional aldehyde derivatization chemistries.

The invention will now be particularly described by way of examples with reference to the accompanying drawings in which:
Figure 1 shows the FT-IR spectra of: (a) 3-vinylbenzaldehyde monomer; (b) 3-vinylbenzaldehyde pulsed plasma polymer (t*ₒₙ* = 50 µs, t*_{off}* = 4 ms); and (c) SW continuous wave 3-vinylbenzaldehyde plasma polymer.
Figure 2 shows the Fluorescence Intensity Variation with pulsed plasma on-time of: (a) Cy5 Tagged DNA immobilized onto 3-vinylbenzaldehyde plasma polymer surfaces (1% excitation laser intensity); and (b) the hybridisation of Cy5 tagged DNA to surface immobilised DNA strands (10% excitation laser intensity) on a 3-vinylbenzaldehyde plasma polymer surface (*Pp* = 40 W and *t_{off}* = 4 ms).
Figure 3 shows Cy5 Tagged DNA hybridized to spots of surface immobilised DNA on a 3-vinylbenzaldehyde pulsed plasma polymer surface (t*ₒₙ* = 50 µs, t*_{off}* = 4 ms).
Figure 4 shows Cy5 tagged ssDNA immobilised onto 3-vinylbenzaldehyde functionalised treated polystyrene beads. Examined by (a) fluorescence microscopy, and (b) visible microscopy.
Figure 5 shows amine terminated Cy5-tagged DNA spatially addressed onto a 10-undecenal pulsed plasma polymer surface (t*ₒₙ* = 15 µs, t*_{off}*= 20 ms).
Figure 6 shows the XPS spectra of (a) 3-vinylbenzaldehyde pulsed plasma polymer and (b) the 3-vinylbenzaldehyde plasma polymer following reaction with 1M ammonium hydroxide and 0.1M silver nitrate.

Scheme 1 shows a method of the invention for enabling DNA hybridisation on surfaces: (a) Aldehyde surface functionalisation by pulsed plasma polymerisation of 3-vinylbenzaldehyde, (b) Immobilisation of amine terminated ssDNA onto the pulsed plasma polymer surface by Schiff-base chemistry, and (c) Hybridisation of complimentary Cy5 tagged ssDNA to surface immobilised ssDNA.

The following examples are intended to illustrate the present invention but are not intended to limit the same:

### Example 1

Plasma polymerization of 3-vinylbenzaldehyde (Aldrich, 97%, H₂C = CH(C₆H₄)CHO, purified by several freeze-pump-thaw cycles) was carried out in an electrodeless cylindrical glass reactor (5 cm diameter, 520 cm³ volume, base pressure 3 x 10⁻² mbar, leak rate = 1 x 10⁻⁹ mol s⁻¹) enclosed in a Faraday Cage. The chamber was fitted with a gas inlet, a thermocouple pressure gauge and a 30 L min⁻¹ two-stage rotary pump connected to a liquid nitrogen cold trap. All joints were grease free. An externally wound 4 mm diameter copper coil spanned 8 -15 cm from the gas inlet with 9 turns.

The output impedance of a 13.56 MHz RF power supply was matched to the partially ionized gas load with an L-C matching network. In the case of pulsed plasma deposition, the RF source was triggered from an external signal generator, and the pulse shape monitored with a cathode ray oscilloscope. The reactor was cleaned by scrubbing with detergent, rinsing in water, propan-2-ol and drying in an oven. The reactor was further cleaned with a 0.2 mbar air plasma operating at 40 W for a period of 30 min. Each substrate was sonically cleaned in a 50:50 mixture of cyclohexane and propan-2-ol for 10 min and then placed into the centre of the reactor on a flat glass plate.

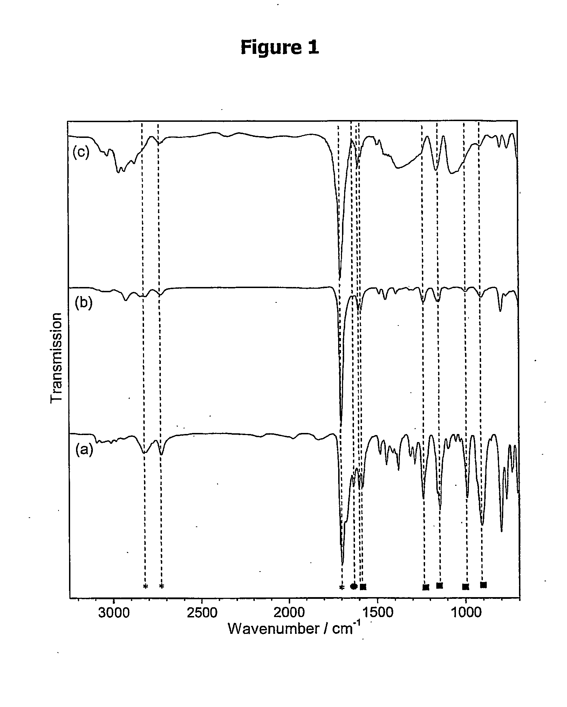

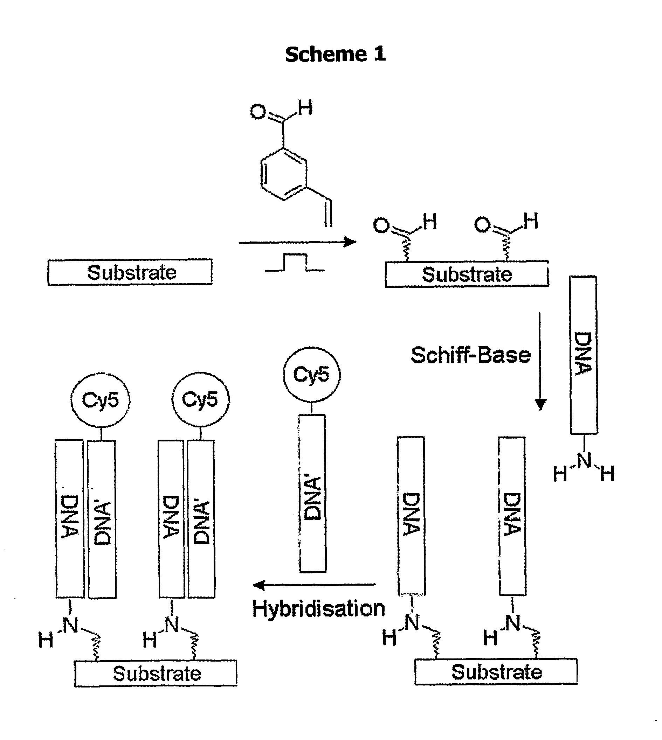

A comparison of the infrared spectra obtained from low power (5 W) continuous wave and pulsed plasma deposited films shows that the distinctive aldehyde CHO stretch at 2815 cm⁻¹ and 2723 cm⁻¹ and the aldehyde C=O stretch at 1695 cm⁻¹ are markedly reduced and broadened for the former, relative to the C-H stretches in the 2836-3030cm⁻¹ region, Figure 1 and Table 1. The C=C stretch at 1650cm⁻¹ associated with 3-vinylbenzaldehyde monomer is absent. Bands from meta-substituted phenyl ring in the fingerprint region of the pulsed plasma polymer are also clearly discernible.

**Table 1**

| The Assignment of 3-vinylbenzaldehyde FT-IR absorbances. | |
|---|---|
| Wavenumber (cm⁻¹) | Assignment |
| 2836-3030 | C-H stretches |
| 2815 | CHO stretch * |
| 2723 | CHO stretch * |
| 1695 | C=O stretch * |
| 1650 | C=C stretch □ |
| 1595 | Di-substituted benzene quadrant stretch |
| 1581 | Di-substituted benzene quadrant stretch |
| 1478 | Meta-substituted benzene semicircle stretch |
| 1446 | Meta-substituted benzene semicircle stretch |
| 1410 | C=CH₂ scissors deformation |
| 1386 | Aldehyde CH rock |
| 1309 | C=CH rock |
| 1145 | Meta ring stretch |
| 992 | Meta in-phase CH wag |
| 908 | Meta single CH wag |

| | |
|---|---|
| * denotes aldehyde absorbances, ● denotes the polymerizable alkene C=C band in Figure 1. | |

The XPS surface elemental compositions of both the low power (5W) continuous wave and pulsed 3-vinylbenzaldehyde plasma polymers appeared to be in good agreement with the theoretical composition based on the monomer structure, Table 2. Absence of any Si(2p) signal was indicative of a pinhole-free film, whilst the loss of Na(1s) and Cl(2p) signals corresponded to the complete removal of buffer salts during washing.

**Table 2**

| The XPS atomic composition of 3-vinylbenzaldehyde plasma polymers. | | |
|---|---|---|
| | **% Carbon** | **% Oxygen** |
| Theoretical | 90 | 10 |
| Pulsed Plasma Polymer | 89 ±2 | 11 ±2 |
| Continuous Wave Plasma Polymer | 91 ±2 | 9 ±2 |

### Example 2

DNA immobilization to pulsed plasma polymerised 3-vinylbenzaldehyde surfaces entailed immersing 3-vinylbenzaldehyde plasma polymer surfaces, prepared as described in example 1, into 1.0 µmol dm⁻³ of fluorescently tagged oligonucleotide (Sigma-Genosys Ltd., oligonucleotide sequence: 5'-3' AACGATGCACGAGCA, desalted, reverse phase purified with 3' terminal primary amine and 5' terminal Cy5 fluorophore) at 42°C for 16h in saline sodium citrate buffer at pH=4.5 (citric acid 99%, Aldrich; NaCl 99.9%, Sigma). Subsequently 3.5 mg ml⁻¹ NaCN(BH₃) (Aldrich, 99%) was added and the solution gently stirred for 3h. Excess physisorbed probe oligonucleotides were removed by sequential washing in high purity water; saline sodium citrate buffer (SSC, 0.3 M Sodium Citrate, 3M NaCl, pH=7, Sigma) with 1% sodium dodecyl sulphate (Sigma, 10% solution); high purity water; solution of 10% stock SSC buffer in high purity water with 0.1% (w/v) sodium dodecyl sulphate; and finally, high purity water; 5% stock SSC buffer in high purity water; high purity water.

Fluorescently labelled oligonucleotides attached to the surface were identified using a fluorescence microscope (Dilor Labram) fitted with a 10x lens, and a 20 mW HeNe laser (632.817 nm wavelength) which corresponds to the excitation range of the Cy5 fluorophore. A polarization of 500:1 was chosen, and the laser beam passed through a diffraction grating of 1800 lines mm⁻¹. Due to the high fluorescence of some surfaces, a filter permitting only 1% laser energy transmission was used unless otherwise stated. A low-level fluorescence background was present for the glass slides, with a broad shallow peak at approximately 2800 cm⁻¹.

For the hybridization studies, an oligonucleotide (sequence: 5'-3' GCTTATCGAGCTTTC, desalted, reverse phase purified with 5' terminal primary amine, Sigma-Genosys Ltd.) was attached onto 3-vinylbenzaldehyde plasma polymer surfaces as described above. These surfaces were then immersed in a solution of 50% pre-hybridization solution (Sigma, from 2x concentrate) and 50% formamide (Sigma, molecular biology grade) for 1 h. The treated polymer surface was removed from solution, rinsed in high purity H₂O and immersed in a 50% high purity H₂O / 50% hybridization solution (Sigma, from 2 x concentrate), with 200 nM of hybridising oligonucletide (sequence: 5'-3' GAAAGCTCGATAAGC, desalted, reverse phase purified with 5' terminal Cy5 fluorophore, Sigma-Genosys Ltd.) at 20 °C for 1 h. These hybridized surfaces were then washed sequentially as described previously.

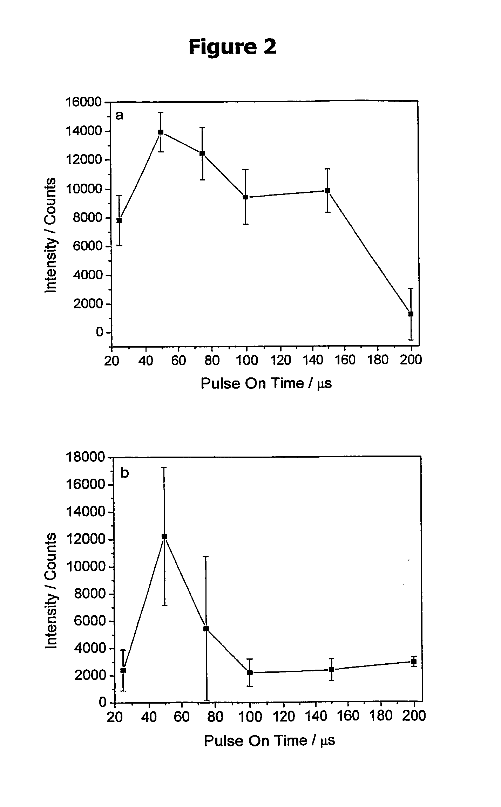

Pulsed plasma deposition conditions corresponding to a duty cycle with t*ₒₙ* = 50 µs were shown by fluorescence intensity measurements to be efficient for both the immobilization of oligonucleotides and the subsequent hybridization of surface immobilized oligonucleotides, Figure 2.

### Example 3

Similarly to the procedure described above, oligonucleotides were spatially addressed onto 3-vinylbenzaldehyde pulsed plasma polymer coated glass microscope slides using a robotic spotter (Genepak).
Probe solutions were placed in a 384-well plate and the robot used a stainless steel pin to pick up and spot solution onto the functionalized slides. Typically, 4 identical 500 µm print pitch arrays were constructed onto the slide, using a pin pick-up time of 1 s and a 0.01 s dwell time. The spotted arrays were incubated in an oven at 42 °C over a saturated solution of K₂SO₄ (96% relative humidity) for 16h and cleaned as outlined above in order to remove non-covalently-bound material.

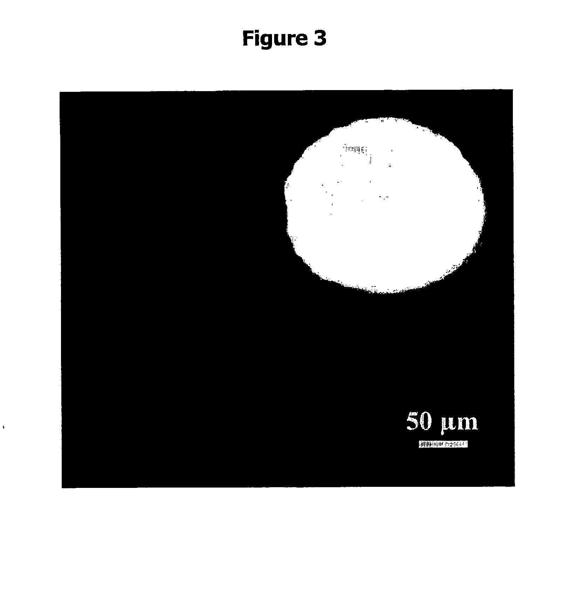

On examination, an array of DNA modified regions was clearly visible, Figure 3.

### Example 4

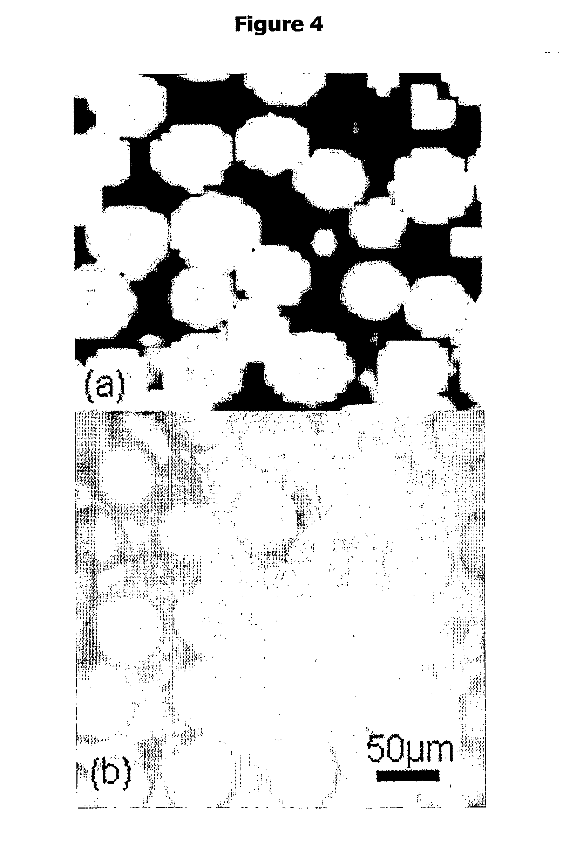

3-vinylbenzaldehyde was deposited onto polystyrene beads (Biosearch Technologies, Inc.) as described above. These aldehyde functionalised beads were then derivatised with fluorescently tagged DNA strands as described above. The derivatisation was confirmed by fluorescence microscopy, Figure 4.

### Example 5

The methodology of Example 1 was utilised to effect the polymerisation of undecenal (Aldrich, +99%).

The XPS surface elemental compositions of the pulsed 10-undecenal plasma polymer (tₒₙ = 10 µs, t_{off} = 20 ms) appeared to be in good agreement with the theoretical composition based on the monomer structure. Low power (3W) continuous wave polymerisation resulted in a marked increase in oxygenation of the surface, Table 3.

**Table 3**

| The XPS atomic composition of 10-undecenal plasma polymers. | | |
|---|---|---|
| | **% Carbon** | **% Oxygen** |
| Theoretical | 91.7 | 8.3 |
| Pulsed Plasma Polymer | 92.0 | 8.0 |
| Continuous Wave Plasma Polymer | 86.3 | 13.7 |

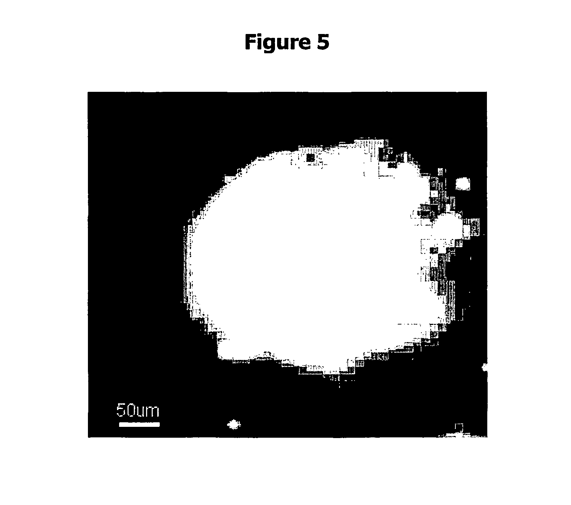

This surface was suitable for derivatisation by amine modification as in Example 3. The successful attachment of fluorescently tagged amine-terminated DNA to a pulsed plasma polymerized undecanal surface is shown in Figure 5.

### Example 6

Silver deposition was performed on a pulsed plasma polymerised 3-vinylbenzaldehyde surface. This firstly comprised plasma deposition as described in Example 1, followed by immersion in an aqueous solution of 1.0 M ammonium hydroxide (Aldrich) and 0.1M silver nitrate (Apollo Scientific) for 24 hours. Samples were then washed under gentle stirring in high purity water for 16 hours before immersion in a fresh water solution for 7 days.

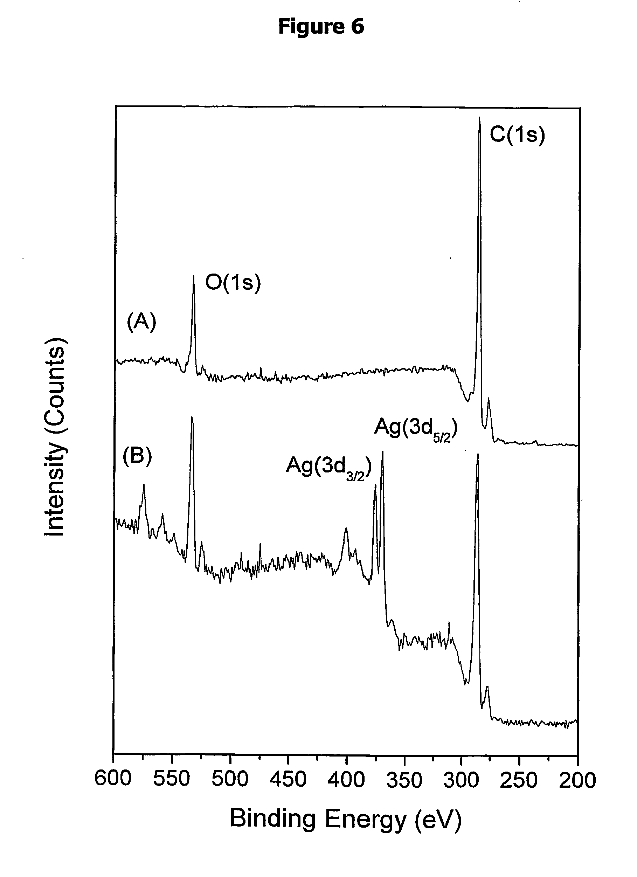

XPS of the plasma polymer surface prior to treatment showed only carbon and nitrogen present on the surface, Figure 6a. After silver deposition, XPS peaks at 374 eV and 368 eV were observed, corresponding to the Ag(3d_{3/2}) and Ag(3d_{5/2}) levels respectively. The intensity of the C(1s) envelope was also reduced relative to the O(1s) envelope, due to the expected oxidation of surface aldehyde functionality during the reaction, Figure 6a.

## Claims

1. A method for applying a reactive aldehyde containing coating to a substrate, said method including subjecting said substrate to a plasma discharge in the presence of a compound of formula (I) 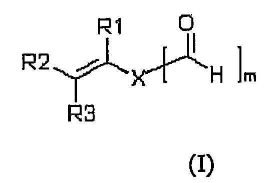 Where X is an optionally substituted straight or branched alkylene chain(s) or aryl group(s); R¹, R² or R³ are optionally hydrogen, substituted hydrocarbyl or heterocyclic groups, and m is an integer greater than 0.

2. A method according to claim 1 wherein the reactive aldehyde containing compound is a compound of formula (II) 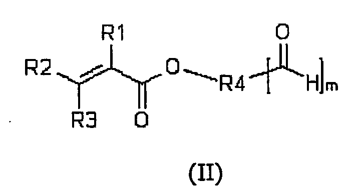 Where R⁴ is an optionally substituted hydrocarbyl or heterocyclic group.

3. A method according to claim 2 wherein the compound of formula (II) is a compound of formula (III) 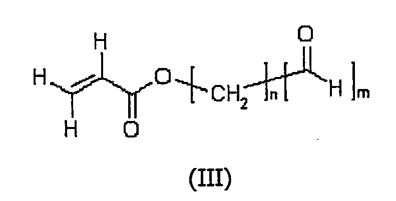 where n=1-20.

4. A method according to claim 2 wherein the compound of formula (II) is a compound of formula (IIIa) 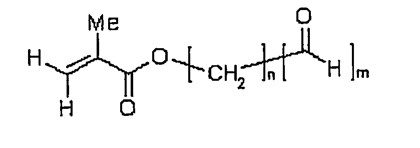 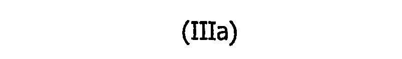 where n=1-20.

5. A method according to claim 1 wherein the aldehyde containing compound of formula (I) is a compound of formula (IV)

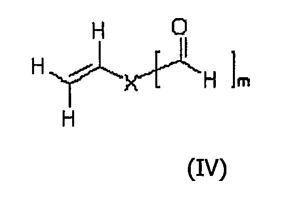

6. A method according to claim 5 wherein the compound of formula (IV) is a compound of formula (V)

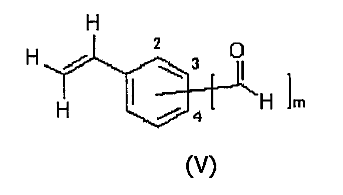

7. A method according to claim 5 wherein the compound of formula (IV) is a compound of formula (Va) 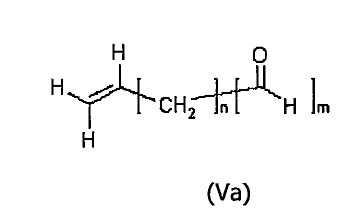 where n=1-20.

8. A method according to claim 1 wherein the plasma discharge is pulsed.

9. A method according to claim 8 wherein the average power of the pulsed plasma discharge is less than 0.05 W/cm³.

10. A method according to claim 8 wherein the pulsed plasma discharge is applied such that the power is on for from 10 µs to 100 µs, and off for from 1000 µs to 20000 µs.

11. A method according to claim 8 wherein the pulsed plasma discharge is applied such that the pulsing regime changes in a controlled manner throughout the course of a single coating deposition.

12. A method according to claim 1 wherein the plasma discharge contains the compound of formula (I) in the absence of any other material.

13. A method according to claim 1 wherein additional materials to the compound of formula (I) are added to the plasma discharge.

14. A method according to claim 13 wherein said additional materials are inert and are not incorporated within the reactive aldehyde containing product coating.

15. A method according to claim 13 wherein said additional materials are non-inert and possess the capability to modify and/or be incorporated into the reactive aldehyde containing product coating.

16. A method according to claim 15 wherein the use of said non-inert additional materials results in a copolymer coating that contains reactive aldehyde functionality.

17. A method according to any preceding claim wherein the introduction of the compound of formula (I) and/or any additional materials into the plasma discharge is pulsed.

18. A method according to any preceding claim wherein the compound of formula (I) and/or any additional materials are introduced into the plasma discharge in the form of atomised liquid droplets.

19. A method according to claim 1 wherein the means for applying the coating is a reel-to-reel equipped plasma deposition apparatus.

20. A method according to claim 1 wherein the plasma deposition chamber is heated.

21. A substrate having an aldehyde containing coating thereon, obtained by a process according to any one of the preceding claims.

22. A method according to any one of the preceding claims which further includes the step of derivatization or reaction of the aldehyde groups after the deposition of the coating.

23. A method according to claim 22 wherein the step of the derivatization or reaction of the aldehyde groups is performed with an amine group.

24. A method according to claim 23 wherein a solution of said amine is contacted with the surface under conditions in which the amine functionality reacts with aldehyde groups on the surface.

25. A method for the immobilisation of an amine-containing reagent at a surface, said method including the application of a reactive aldehyde containing coating to said surface by a method according to any preceding claims, and then contacting the surface with a solution of said amine-containing agent under conditions such that the amine group reacts with the aldehyde groups.

26. A method according to claim 25 wherein the immobilisation of the amine solution is spatially addressed onto the reactive aldehyde containing surface, such that amine immobilisation occurs only in given spatial locations.

27. A method according to claims 23 to 26 in which the amine is an amine-terminated biomolecule.

28. A method according to claim 27 wherein the modified surface is utilised for DNA hybridisation.

29. A method according to claim 22 wherein a solution of a silver containing salt reacts with surface aldehyde groups, resulting in silver metallization of the polymer surface.

30. A method according to claim 29 wherein the Tollens reaction is used to generate metallic silver on the reactive aldehyde surface.

31. A method according to any of the preceding claims wherein the substrate is any or any combination of metal, glass, semiconductor, ceramic, polymer, woven or non-woven fibres, natural fibres, cellulosic material or powder.

32. A method according to claim 1 wherein R¹, R² and/or R³ include fluoro, chloro, bromo and/or iodo substituents.

33. A method according to claim 3 wherein the compound of formula III, where m=1 and n=1, is ethylaldehyde acrylate.

34. A method according to claim 6 wherein the compound of formula V is 3-vinylbenzaldehyde.

35. A method according to claim 7 wherein the compound of formula Va, where m=1 and n=8, is 10-undecenal.

36. A method according to claim 9 wherein the average power of the pulsed plasma discharge is less than 0.0025 W/cm³.

## Patentansprüche

1. Verfahren zum Aufbringen einer reaktiven aldehydhaltigen Beschichtung auf ein Substrat, wobei das genannte Verfahren das Aussetzen des genannten Substrats einer Plasmaentladung in Anwesenheit einer Verbindung der folgenden Formel (I) beinhaltet 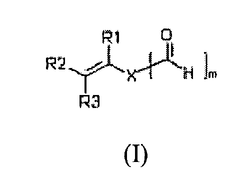 wobei X (eine) optional substituierte gerade oder verzweigte Alkylenkette(n) oder Arylgruppe(n) ist; R¹, R² oder R³ optional Wasserstoff-, substituierte Hydrocarbyl- oder heterozyklische Gruppen sind und m eine ganze Zahl größer als 0 ist.

2. Verfahren nach Anspruch 1, wobei die reaktive aldehydhaltige Verbindung eine Verbindung der Formel (II) ist 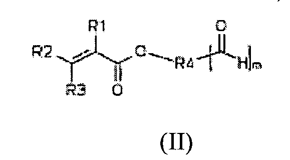 wobei R⁴ eine optional substituierte Hydrocarbyl- oder heterozyklische Gruppe ist.

3. Verfahren nach Anspruch 2, wobei die Verbindung der Formel (II) eine Verbindung der Formel (III) ist 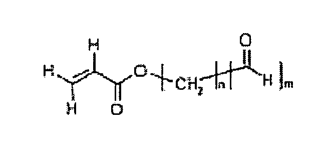 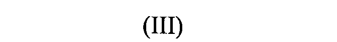 wobei n = 1-20 ist.

4. Verfahren nach Anspruch 2, wobei die Verbindung der Formel (II) eine Verbindung der Formel (IIIa) ist 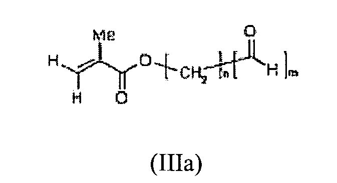 wobei n = 1-20 ist.

5. Verfahren nach Anspruch 1, wobei die aldehydhaltige Verbindung der Formel (I) eine Verbindung der Formel (IV) ist

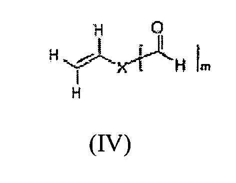

6. Verfahren nach Anspruch 5, wobei die Verbindung der Formel (IV) eine Verbindung der Formel (V) ist

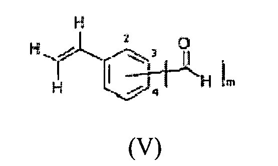

7. Verfahren nach Anspruch 5, wobei die Verbindung der Formel (IV) eine Verbindung der Formel (Va) ist 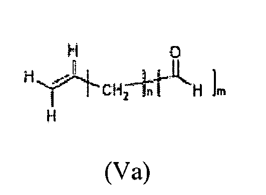 wobei n = 1-20 ist.

8. Verfahren nach Anspruch 1, wobei die Plasmaentladung gepulst ist.

9. Verfahren nach Anspruch 8, wobei die durchschnittliche Leistung der gepulsten Plasmaentladung weniger als 0,05 W/cm³ beträgt.

10. Verfahren nach Anspruch 8, wobei die gepulste Plasmaentladung so appliziert wird, dass sie 10 µs bis 100 µs lang eingeschaltet und 1000 µs bis 20.000 µs lang ausgeschaltet ist.

11. Verfahren nach Anspruch 8, wobei die gepulste Plasmaentladung so erfolgt, dass sich die Pulsierroutine in einer kontrollierten Art und Weise im Laufe einer einzelnen Beschichtungsdeposition ändert.

12. Verfahren nach Anspruch 1, wobei die Plasmaentladung die Verbindung der Formel (I) in Abwesenheit eines beliebigen anderen Materials beinhaltet.

13. Verfahren nach Anspruch 1, wobei zusätzliche Materialien zur Verbindung der Formel (I) zur Plasmaentladung zugegeben werden.

14. Verfahren nach Anspruch 13, wobei die genannten zusätzlichen Materialien inert sind und nicht in der reaktiven aldehydhaltigen Produktbeschichtung aufgenommen werden.

15. Verfahren nach Anspruch 13, wobei die genannten zusätzlichen Materialien nicht inert sind und die Fähigkeit haben, die reaktive aldehydhaltige Produktbeschichtung zu modifizieren und/oder in ihr aufgenommen zu werden.

16. Verfahren nach Anspruch 15, wobei die Verwendung der genannten nicht inerten zusätzlichen Materialien in einer Copolymerbeschichtung resultiert, die reaktive Aldehydfunktionalität umfasst.

17. Verfahren nach einem der vorherigen Ansprüche, wobei die Einführung der Verbindung der Formel (I) und/oder beliebiger zusätzlicher Materialien in die Plasmaentladung gepulst wird.

18. Verfahren nach einem der vorherigen Ansprüche, wobei die Verbindung der Formel (I) und/oder beliebige zusätzliche Materialien in die Plasmaentladung in Form zerstäubter Flüssigkeitströpfchen eingeführt wird/werden.

19. Verfahren nach Anspruch 1, wobei das Mittel zum Aufbringen der Beschichtung eine von Trommel auf Trommel arbeitende Plasmadepositionsvorrichtung ist.

20. Verfahren nach Anspruch 1, wobei die Plasmadepositionskammer erhitzt wird.

21. Substrat mit einer aldehydhaltigen Beschichtung darauf, die durch einen Prozess nach einem der vorherigen Ansprüche erhalten wird.

22. Verfahren nach einem der vorherigen Ansprüche, das ferner den Schritt des Derivatisierens oder Reagierens der Aldehydgruppen nach der Deposition der Beschichtung beinhaltet.

23. Verfahren nach Anspruch 22, wobei der Schritt des Derivatisierens oder Reagierens der Aldehydgruppen mit einer Amingruppe ausgeführt wird.

24. Verfahren nach Anspruch 23, wobei eine Lösung des genannten Amins mit der Oberfläche unter Bedingungen in Kontakt gebracht wird, bei denen die Aminfunktionalität mit Aldehydgruppen auf der Oberfläche reagiert.

25. Verfahren zum Immobilisieren eines aminhaltigen Reagens an einer Oberfläche, wobei das genannte Verfahren das Aufbringen einer reaktiven aldehydhaltigen Beschichtung auf die genannte Oberfläche mit einem Verfahren gemäß einem der vorherigen Ansprüche und dann das Inkontaktbringen der Oberfläche mit einer Lösung des genannten aminhaltigen Agens unter solchen Bedingungen beinhaltet, dass die Amingruppe mit den Aldehydgruppen reagiert.

26. Verfahren nach Anspruch 25, wobei die Immobilisation der Aminlösung auf der reaktiven aldehydhaltigen Oberfläche räumlich erfolgt, so dass die Aminimmobilisation nur in bestimmten räumlichen Orten stattfindet.

27. Verfahren nach den Ansprüchen 23 bis 26, wobei das Amin ein aminterminiertes Biomolekül ist.

28. Verfahren nach Anspruch 27, wobei die modifizierte Oberfläche zur DNA-Hybridisierung genutzt wird.

29. Verfahren nach Anspruch 22, wobei eine Lösung eines silberhaltigen Salzes mit Oberflächen-Aldehydgruppen reagiert, was in einer Silbermetallisierung der Polymeroberfläche resultiert.

30. Verfahren nach Anspruch 29, wobei die Tollens-Reaktion zum Erzeugen von metallischem Silber auf der reaktiven Aldehydoberfläche verwendet wird.

31. Verfahren nach einem der vorherigen Ansprüche, wobei das Substrat ein beliebiges oder eine beliebige Kombination der Folgenden ist: Metall, Glas, Halbleiter, Keramik, Polymer, gewebte und ungewebte Fasern, natürliche Fasern, Cellulosematerial oder Pulver.

32. Verfahren nach Anspruch 1, wobei R¹, R² und/oder R³ Fluor-, Chlor-, Brom-und/oder Iod-Substituenten beinhalten.

33. Verfahren nach Anspruch 3, wobei die Verbindung der Formel III, bei der m = 1 und n = 1 sind, Ethylaldehydacrylat ist.

34. Verfahren nach Anspruch 6, wobei die Verbindung der Formel V 3-Vinylbenzaldehyd ist.

35. Verfahren nach Anspruch 7, wobei die Verbindung der Formel Va, bei der m = 1 und n = 8 sind, 10-Undecenal ist.

36. Verfahren nach Anspruch 9, wobei die durchschnittliche Leistung der gepulsten Plasmaentladung weniger als 0,0025 W/cm³ beträgt.

## Revendications

1. Méthode d'application, à un substrat, d'un revêtement contenant un aldéhyde, ladite méthode comprenant la soumission dudit substrat à une décharge plasma en présence d'un composant ayant la formule (I) 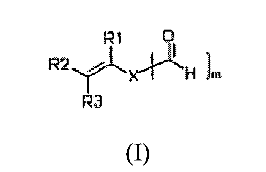 dans laquelle X est une ou plusieurs chaînes alkylènes ou un ou plusieurs groupes aryles linéaires ou ramifiés, éventuellement substitués ; R¹, R² ou R³ sont des groupes hydrocarbyles ou hétérocycliques éventuellement substitués par l'hydrogène, et m est un nombre entier supérieur à 0.

2. Méthode selon la revendication 1, selon laquelle le composé contenant l'aldéhyde réactif est un composé ayant la formule (II) 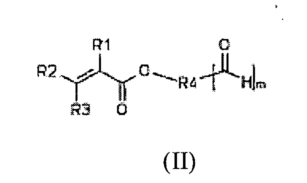 dans laquelle R⁴ est un groupe hydrocarbyle ou hétérocyclique éventuellement substitué.

3. Méthode selon la revendication 2, selon laquelle le composé de la formule (II) est un composé de la formule 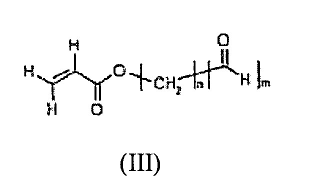 dans laquelle n=1 à 20.

4. Méthode selon la revendication 2, selon laquelle le composé de la formule (II) est un composé ayant la formule (IIIa) 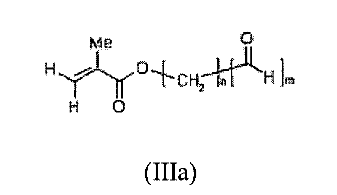 dans laquelle n=1 à 20.

5. Méthode selon la revendication 1, selon laquelle le composé contenant de l'aldéhyde de la formule (I) est un composé ayant la formule (IV)

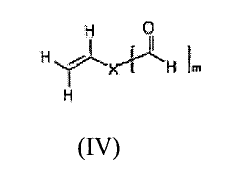

6. Méthode selon la revendication 5, selon laquelle le composé de la formule (IV) est un composé ayant la formule (V)

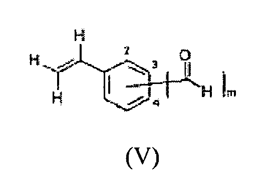

7. Méthode selon la revendication 5, selon laquelle le composé de la formule (IV) est un composé ayant la formule (Va) 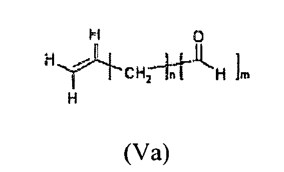 dans laquelle n=1 à 20.

8. Méthode selon la revendication 1, selon laquelle la décharge plasma est pulsée.

9. Méthode selon la revendication 8, selon laquelle la puissance moyenne de la décharge plasma pulsée est inférieure à 0,05 W/cm³.

10. Méthode selon la revendication 8, selon laquelle la décharge plasma pulsée est activée pendant 10 à 100 µs et désactivée pendant 1000 à 20000 µs.

11. Méthode selon la revendication 8, selon laquelle la décharge plasma pulsée est appliquée de telle sorte que le régime des pulsations change de manière contrôlée pendant l'évolution de chacun des dépôts de revêtement.

12. Méthode selon la revendication 1, selon laquelle la décharge plasma contient le composé de la formule (I) en l'absence de tout autre matériau.

13. Méthode selon la revendication 1, selon laquelle des matériaux supplémentaires au composé de la formule (I) sont ajoutés à la décharge plasma.

14. Méthode selon la revendication 13, selon laquelle lesdits matériaux supplémentaires sont inertes et ne sont pas incorporés dans le produit de revêtement contenant l'aldéhyde réactif.

15. Méthode selon la revendication 13, selon laquelle lesdits matériaux supplémentaires ne sont pas inertes et sont capables de modifier le produit de revêtement contenant l'aldéhyde réactif et/ou d'être incorporés dans celui-ci.

16. Méthode selon la revendication 15, selon laquelle l'utilisation desdits matériaux non inertes résulte en un revêtement copolymère qui contient la fonction aldéhyde réactive.

17. Méthode selon l'une quelconque des revendications précédentes selon laquelle l'introduction, dans la décharge plasma, du composé de la formule (I) et/ou d'éventuels matériaux supplémentaires, est pulsée.

18. Méthode selon l'une quelconque des revendications précédentes, selon laquelle le composé de la formule (I) et/ou d'éventuels matériaux supplémentaires sont introduits dans la décharge plasma sous forme de gouttelettes de liquide atomisé.

19. Méthode selon la revendication 1, selon laquelle le moyen d'application du revêtement est un appareil de dépôt de plasma à bobines débitrices/réceptrices.

20. Méthode selon la revendication 1, selon laquelle la chambre de dépôt de plasma est chauffée.

21. Substrat recouvert d'un revêtement contenant un aldéhyde, obtenu par un procédé conforme à l'une quelconque des revendications précédentes.

22. Méthode selon l'une quelconque des revendications précédentes, qui comprend de plus l'étape de dérivatisation ou de réaction des groupes aldéhydes après le dépôt du revêtement.

23. Méthode selon la revendication 22, selon laquelle l'étape de dérivatisation ou de réaction des groupes aldéhydes est effectuée avec un groupe aminé.

24. Méthode selon la revendication 23, selon laquelle une solution de ladite amine est mise en contact avec la surface dans des conditions dans lesquelles la fonction amine réagit avec les groupes aldéhydes sur la surface.

25. Méthode pour l'immobilisation, sur une surface, d'un réactif contenant une amine, ladite méthode comprenant l'application, à ladite surface, d'un revêtement contenant un aldéhyde réactif par une méthode selon l'une quelconque des revendications précédentes, puis la mise en contact de la surface avec une solution dudit agent contenant une amine dans des conditions telles que le groupe aminé réagit avec les groupes aldéhydes.

26. Méthode selon la revendication 25, selon laquelle l'immobilisation de la solution aminée est spatialement dirigée sur la surface contenant l'aldéhyde réactif de sorte que l'immobilisation de l'amine ne survient que dans des emplacements spatiaux donnés.

27. Méthode selon les revendications 23 à 26, selon laquelle l'amine est une biomolécule à terminaison -amine.

28. Méthode selon la revendication 27, selon laquelle la surface modifiée est utilisée pour l'hybridation de l'ADN,

29. Méthode selon la revendication 22, selon laquelle une solution d'un sel contenant de l'argent réagit avec les groupes aldéhydes de la surface, ce qui résulte en la métallisation à l'argent de la surface du polymère.

30. Méthode selon la revendication 29, selon laquelle la réaction de Tollens est utilisée pour la génération d'argent métal sur la surface de l'aldéhyde réactif.

31. Méthode selon l'une quelconque des revendications précédentes, selon laquelle le substrat peut être métal, verre, semiconducteur, céramique, polymère, fibre tissée ou non tissée, fibre naturelle, matériau cellulosique ou poudre, ou une quelconque combinaison de ceux-ci.

32. Méthode selon laquelle R¹, R² et/ou R³ incluent les substituants fluoro, chloro, bromo et/ou iodo.

33. Méthode selon la revendication 3, selon laquelle le composé de la formule III, dans laquelle m=1 et n=1, est l'acrylate d'éthylaldéhyde.

34. Méthode selon la revendication 6, selon laquelle le composé de la formule V est 3-vinylbenzaldéhyde.

35. Méthode selon la revendication 7, selon laquelle le composé de la formule Va, dans laquelle m=1 et n=8, est 10-undecenal.

36. Méthode selon la revendication 9, selon laquelle la puissance moyenne de la décharge plasma pulsée est inférieure à 0,0025 W/cm³.
